# FASCICULE DE BREVET EUROPEEN

(11) **EP 2 186 479 B1**
(45) Date de publication et mention de la délivrance du brevet: **15.04.2020**
(21) Numéro de dépôt: 09161145.9
(22) Date de dépôt: 26.05.2009
(51) Int. Cl.: A61B 5/107, A61B 6/02

(54) **Mesure de grandeurs geometriques intrinseques a un systeme anatomique.**
Messung intrinsischer geometrischer Größenmaße eines anatomischen Systems
Measurement of geometrical magnitudes intrinsic in an anatomical system.

(30) Priorité: 24.10.2008 FR 0857245
(43) Date de publication de la demande: 19.05.2010
(73) Titulaire: EOS Imaging, 75011 Paris (FR)
(72) Inventeur: Saint Felix, Didier, 91370, VERRIERE LE BUISSON (FR); Di Monda, Richard, MARIETTA - USA, GA GA 30068 (US)
(74) Mandataire: Plasseraud IP

(56) Documents cités:
- EP-A- 1 168 249
- WO-A-02/062249
- FR-A1- 2 856 170

## Description

L'invention se rapporte à la mesure de grandeurs géométriques intrinsèques à un système anatomique.

Un praticien, par exemple un radiologue, un chirurgien, ou autre, peut en effet utiliser des grandeurs géométriques, par exemple une longueur d'un os, un angle d'une scoliose, ou autre, pour établir un diagnostic.

Il est connu d'effectuer une radiographie du système ostéo-articulaire d'un patient et de mesurer des grandeurs géométriques sur l'image obtenue.

Toutefois, la valeur mesurée est entachée d'erreurs causées par le processus de projection des rayons sur le plan du capteur. Cette erreur est susceptible de varier selon la position du patient dans le référentiel de l'installation d'imagerie. Le document EP 1 168 249 A1 décrit un procédé d'imagerie radiographique pour la reconstruction tridimensionnelle à faible dose d'irradiation du squelette d'un patient à partir de deux images radiographiques perpendiculaires et d'une connaissance a priori de ce squelette.

L'invention vient remédier à cet inconvénient en proposant un procédé de mesure de grandeurs géométriques intrinsèques à un système anatomique, selon la revendication 1.

Ainsi, les grandeurs géométriques mesurées sont intrinsèques au système anatomique, typiquement un système ostéo-articulaire. Ces grandeurs sont donc indépendantes de la position du patient dans le référentiel de l'installation d'imagerie. Si une acquisition d'images doit être répétée, afin par exemple de suivre l'évolution d'une grandeur géométrique donnée, les grandeurs géométriques mesurées ne sont pas affectées par les variations de position du patient d'une acquisition à l'autre.

Ce procédé est relativement simple pour l'utilisateur et relativement simple à mettre en œuvre.

Les images stéréoscopiques sont réalisées à partir de deux points de vue différents. On peut prévoir d'utiliser un capteur pour chaque point de vue de façon à acquérir simultanément les deux images stéréoscopiques. En variante, on peut utiliser un bras portant une source et un détecteur, apte à tourner autour du patient (« C-arm » en anglais).

Les images stéréoscopiques sont ici géométriquement calibrées, c'est-à-dire que les deux images stéréoscopiques peuvent être à la même échelle, ou bien que les deux images sont chacune à une échelle particulière, des données d'échelles permettant de passer de l'échelle de chacune des deux images à l'échelle de l'autre des deux images.

Les données d'échelles peuvent par exemple comprendre, pour chacune des images, une valeur d'échelle de l'image permettant de déterminer une dimension d'un objet représenté sur l'image à partir de la dimension de la représentation de cet objet sur l'image. Alternativement, les données d'échelle peuvent simplement comprendre un ratio entre les valeurs d'échelle de chacune des images, ce qui peut être suffisant pour mesurer des grandeurs géométriques de type angle.

Les deux images bidimensionnelles stéréoscopiques dudit système ostéo-articulaire (ou l'une seulement de ces deux images) peuvent être obtenues par deux détecteurs respectifs. Il peut par exemple s'agir d'images obtenues par rayons X, ultrasons, des photographies, ou autre.

Alternativement, les deux images (ou l'une seulement de ces deux images) peuvent être obtenues par deux projections respectives d'un modèle tridimensionnel du système ostéo-articulaire du patient selon des directions distinctes sur des plans virtuels simulant des plans d'acquisition. Le modèle tridimensionnel peut par exemple être un modèle reconstruit, par exemple selon le procédé décrit dans le document EP1168249. Les images peuvent par exemple être obtenues par projections géométriques des contours du modèle, ce qui permet d'obtenir des images relativement précises, ou bien encore par simulation de rayons X à partir du modèle, ce qui permet d'obtenir des images ressemblant à des radiographies, c'est-à-dire un type d'images auquel le praticien est habitué, ou bien encore par une autre projection.

On peut trouver plusieurs applications à la mesure des grandeurs géométriques à partir de deux images stéréoscopiques obtenues à partir d'un modèle tridimensionnel (3D). Cette mesure peut par exemple permettre de valider le modèle 3D. Egalement, on peut choisir des plans de projections tels que le praticien pourra repérer des points sur les images stéréoscopiques avec une précision relativement élevée.

Le procédé décrit ci-dessus et ci-dessous peut être appliqué à la mesure orthopédique de grandeurs géométriques intrinsèques au système ostéo-articulaire, mais également à la mesure de grandeurs géométriques intrinsèques à d'autres organes, comme les tissus mous visibles sur les images stéréoscopiques.

Outre les jeux de coordonnées bidimensionnelles, les données de repérage peuvent comprendre une indication de la grandeur géométrique que l'utilisateur entend mesurer. On peut en particulier prévoir plusieurs types de grandeurs géométriques, par exemple angle 3 points, angle 4 points, distance, ou autre. Un angle 4 points peut être défini comme un angle entre deux droites de l'espace tridimensionnel définies chacune par deux points de l'espace tridimensionnel.

Si par exemple l'utilisateur choisit de mesurer un angle 3 points, trois jeux de coordonnées bidimensionnelles sont attendus ; deux primitives géométriques tridimensionnelles, ici des droites concourantes en un point, sont déterminées à partir de ces jeux de coordonnées bidimensionnelles, et une valeur d'angle entre ces droites est calculée.

On peut prévoir que les données de repérage comprennent seulement des jeux de coordonnées bidimensionnelles, ou bien seulement des jeux de coordonnées bidimensionnelles et une indication de la grandeur géométrique que l'utilisateur entend mesurer.

On peut également prévoir que les données de repérage comprennent des données de définition de primitives. Ces données de définition de primitive permettent de définir une primitive à partir d'un ou plusieurs jeux de coordonnées bidimensionnelles des données de repérage.

Par exemple, les données de repérage peuvent comprendre deux jeux de coordonnées et des données de définition de primitives comprenant une indication de plan médiateur. Ainsi, à partir de ces données de repérage sont définies une primitive segment comprenant deux points de l'espace 3D correspondant chacun à un jeu de coordonnées, et une primitive de plan médiateur de l'espace 3D contenant les médiatrices de la primitive segment.

Par exemple, les données de repérage peuvent comprendre quatre jeux de coordonnées et des données de définition de primitive comprenant une indication de correction de façon à définir deux droites perpendiculaires dans l'un au moins des plans des images stéréoscopiques. Sont dans un premier temps définies deux primitives de droites comprenant chacune deux points de l'espace 3D respectivement définis par des jeux de coordonnées correspondants des données de repérage. Dans un deuxième temps, l'une de ces deux droites, choisie selon les données de définition de primitive, est déplacée de façon à ce que sa projection dans l'un au moins des plans des images stéréoscopiques forme un angle droit avec la projection dans ce même plan de l'autre droite.

En variante, les données de repérage peuvent comprendre d'autres données, par exemple des distances.

Par « informations de calibration géométrique des images stéréoscopiques », on entend des informations permettant de déduire un point de vue ou une distance capteur-détecteur d'une image à partir du point de vue ou de la distance capteur-détecteur de l'autre image. Par exemple, les informations de calibration géométrique des images stéréoscopiques peuvent comprendre les positions absolues des sources et des détecteurs pour chacune des images. En variante, les informations de calibration géométrique peuvent comprendre une position relative de la source par rapport au détecteur pour chaque image. Selon une autre variante, les informations de calibration peuvent comprendre un ratio entre les valeurs d'échelle de chacune des images, ainsi qu'un angle orienté entre les points de vue des images.

Une primitive géométrique tridimensionnelle peut être décrite par une expression analytique, c'est-à-dire avec un nombre de paramètres limité. La primitive déterminée à l'étape (b) peut comprendre une sphère, une droite, un cylindre, un cône, un pavé.

La primitive géométrique est définie par une partie au moins des données de repérage reçues à l'étape (a), sans connaissance a priori de l'objet représenté sur les images. Au moins certaines valeurs de paramètres décrivant la primitive sont déductibles d'une partie correspondante des données de repérage, en utilisant les informations de calibrage. Par exemple, en utilisant des informations de calibrage géométrique, on peut déterminer une droite dans l'espace tridimensionnel (3D) à partir de deux jeux de coordonnées 2D reçus à l'étape (a). Cette droite 3D peut être décrite par une équation de droite avec un nombre de paramètres relativement réduit.

La grandeur géométrique intrinsèque au système ostéo-articulaire peut être calculée dans l'espace 3D, par exemple un angle dans l'espace 3D, par exemple un angle cervico-diaphysaire, un volume. En variante, la grandeur géométrique peut être mesurée dans un plan.

La grandeur géométrique intrinsèque peut être calculée à partir d'une ou plusieurs primitives. Il peut par exemple s'agir du rayon d'une primitive sphérique, ou bien encore d'une distance entre une primitive point et une primitive droite.

En particulier, la grandeur géométrique peut être mesurée dans un plan d'un repère local, attaché au système ostéo-articulaire, et susceptible d'être défini à partir de données de repérage reçues à l'étape (a).

On prévoit à l'étape (c), de calculer au moins une valeur de grandeur géométrique dans au moins un plan, à partir d'au moins un projeté sur cet au moins un plan d'au moins une primitive géométrique tridimensionnelle déterminée à l'étape (b), cet au moins un plan étant défini à partir de données de repérage reçues à l'étape (a). Ainsi, les grandeurs géométriques, par exemple un angle, ou une aire, sont calculées dans un plan attaché au système ostéo-articulaire plutôt que dans le plan de l'un des détecteurs.

Le (ou les) plan peut être déterminé en utilisant les informations de calibration. Par exemple, l'utilisateur clique sur trois points de chacune des images stéréoscopiques et un plan est défini à partir des trois jeux de coordonnées ainsi reçus.

Ces grandeurs géométriques dans un plan attaché au système ostéo-articulaire du patient peuvent être plus pertinentes pour le praticien que des valeurs directement mesurées dans l'espace 3D. En utilisant un plan ou un repère attaché au système ostéo-articulaire, on peut parvenir à des mesures qui ont plus de sens pour le praticien.

En outre, le praticien peut être habitué à mesurer certaines grandeurs dans un plan, typiquement le plan d'une image radiographique. Aussi il peut être plus confortable pour le praticien d'accéder à des grandeurs dans un plan, en particulier à des fins de comparaison avec des grandeurs géométriques mesurées à partir d'une seule image radiographique, selon un procédé de l'art antérieur.

La grandeur géométrique peut comprendre par exemple un angle dans l'espace 3D, un angle entre des projetés de droites de l'espace 3D sur un plan d'un repère local, un volume, ou une aire.

Avantageusement et de façon non limitative, les données de repérage sont reçues suite à la sélection, par un utilisateur, de points sur les images à l'aide d'une interface utilisateur. L'utilisateur pointe ainsi des points sur chacune des images, et la valeur de grandeur géométrique est obtenue à partir de ces points, sans utiliser de connaissance a priori de l'objet imagé.

Avantageusement et de façon non limitative, la valeur de grandeur géométrique calculée à l'étape (c) est affichée sur un écran, de façon à permettre au praticien de poursuivre son analyse. On peut prévoir que suite à cet affichage, le praticien saisisse de nouvelles données de repérage, de sorte que les étapes (a), (b) et (c), ainsi que l'étape d'affichage, soient répétées. Une telle interactivité peut être intéressante pour le praticien, selon les applications souhaitées.

Selon un autre aspect, il est proposé un programme d'ordinateur comportant des instructions pour la mise en œuvre du procédé exposé ci-dessus, lorsque ces instructions sont exécutées par un processeur.

Selon encore un autre aspect, l'invention a pour objet un dispositif de mesure de grandeurs géométriques intrinsèques à un système anatomique, selon la revendication 10.

Un tel dispositif, par exemple un processeur, un ordinateur relié à un appareil d'acquisition d'images stéréoscopiques, ou autre, permet de mettre en œuvre le procédé exposé ci-dessus. Les moyens de réception peuvent par exemple comprendre un port d'entrée, et les moyens de traitement être intégrés dans un processeur.

Dans la présente description, on entend par système ostéo-articulaire aussi bien une partie du système ostéo-articulaire complet d'un patient, que le système ostéo-articulaire complet.

Le patient peut être humain ou animal.

D'autres particularités et avantages de la présente invention apparaîtront dans la description détaillée ci-après, faite en référence aux dessins annexés sur lesquels:
- la figure 1 montre un exemple de système d'acquisition d'images stéréoscopiques comprenant un exemple de dispositif de mesure orthopédique selon un mode de réalisation de l'invention,
- la figure 2 montre un exemple d'interface graphique affichable par un exemple de dispositif de mesure orthopédique selon un mode de réalisation de l'invention,
- la figure 3 montre un exemple d'interface graphique affichable par un exemple de dispositif de mesure orthopédique selon un mode de réalisation de l'invention,
- la figure 4 montre un exemple d'algorithme d'un procédé selon un mode de réalisation de l'invention.
- La figure 5 illustre une étape de détermination de lieu des possibles, pour un procédé selon un mode de réalisation de l'invention,
- La figure 6 montre un exemple d'algorithme d'un procédé selon un mode de réalisation de l'invention.

Des références identiques désignent des objets identiques ou similaires d'une figure à l'autre.

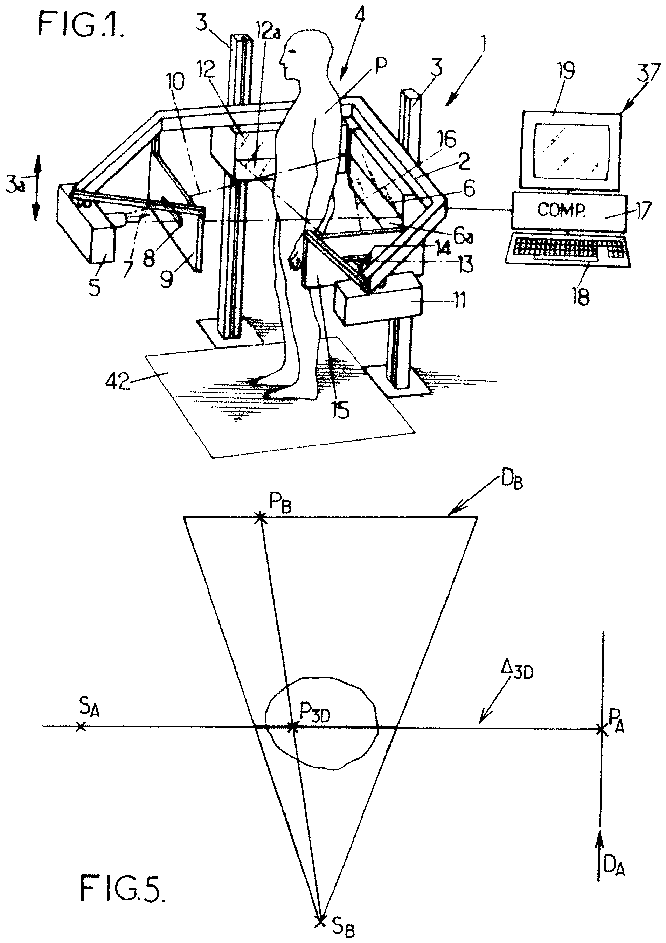

En référence à la figure 1, est représenté un système d'acquisition d'images stéréoscopiques 1 comprenant un support 2 déplaçable selon des guides 3, selon les directions verticales représentées par la double flèche 3a.

Le support 2 définit un champ d'observation 4, dans lequel un patient P peut être placé, par exemple debout, à des fins d'observation du système ostéo-articulaire de ce patient.

Sur le support 2 sont montés une première source 5 à rayons X et un premier détecteur 6 faisant face à la source 5. Ce premier détecteur 6 comprend au moins une ligne horizontale 6a de cellules de détection. Par exemple, le détecteur 6 peut comprendre un détecteur à gaz, par exemple du type de celui décrit dans le document US5959302. Bien entendu, d'autres types de détecteurs peuvent être utilisés dans la cadre de la présente invention.

La source 5 est adaptée pour émettre des rayonnements ionisants, en particulier des rayons X, susceptibles d'être détectés par le détecteur 6, selon une direction de prise de vue 7. Cette direction 7 est sensiblement antéro-postérieure ou postero-antérieure pour le patient P.

Les rayons passent à travers une fente 8 réalisée dans un masque 9, par exemple une plaque métallique, de façon à générer un faisceau horizontal 10 de rayonnement ionisant dans le champ d'observation 4.

Est en outre montée sur le support 2 une deuxième source 11 similaire à la source 5. Un deuxième détecteur 12 similaire au détecteur 6 est monté sur le support 2 de façon à faire face à la source 11. Ce détecteur 12 comprend au moins une ligne horizontale 12a de cellules de détection.

La source 11 est adaptée pour émettre des rayonnements ionisants selon une direction de prise de vue 13 qui est sensiblement latérale par rapport au patient P. Les rayons émis par la source 11 passent à travers une fente horizontale 14 formée dans un masque 15, par exemple une plaque métallique, de sorte qu'un faisceau horizontal 16 de rayons X est générée dans le champ d'observation 4.

Bien entendu, on peut prévoir plus de deux sources et plus de deux détecteurs. En outre, les directions de prises de vue 7 et 13 peuvent ne pas être sensiblement perpendiculaires entre elles. Par exemple, on peut prévoir un angle de 30° entre ces directions de prise de vue. De plus, le plan défini par les directions de prise de vue 7, 13 peut ne pas être parallèle, ni même sensiblement parallèle, au plan du sol. Les directions de prise de vue 7 et 13 peuvent donc être quelconques, pourvu qu'elles permettent effectivement d'obtenir deux images du système ostéo-articulaire du patient et pourvu qu'elles ne soient pas colinéaires entre elles.

Les détecteurs 6, 12 sont raccordés avec un système informatique 37, ou bien avec tout autre system de commande électronique équipé :
- d'une interface utilisateur comprenant une interface d'entrée comprenant un clavier 18, et généralement également une souris (non représentée), et une interface de sortie comportant au moins un écran 19 ;
- un dispositif de mesure orthopédique, par exemple un processeur 17 capable d'exécuter les instructions d'un programme d'ordinateur.

Dans ce mode de réalisation, est fourni un dispositif de reconstruction d'un modèle 3D à partir de deux images stéréoscopiques. Ce dispositif est par exemple intégré dans le processeur 17. Le processeur 17 peut comprendre ou être relié à une mémoire non représentée stockant au moins un modèle a priori de la structure à reconstruire. Ce modèle a priori est établi à partir d'une connaissance a priori de la structure à reconstruire.

L'ordinateur 37 peut être raccordé à des moyens motorisés contenus dans le guide 3, et aux sources 5, 11, de façon à commander le déplacement vertical du support 2 et l'émission de rayons X.

Lors d'une acquisition d'un couple d'images stéréoscopiques, le support 2 est déplacé verticalement, de façon à couvrir une partie du système ostéo-articulaire relativement étendue.

Un tel système 1 permet ainsi d'obtenir deux images stéréoscopiques, acquises simultanément, du système ostéo-articulaire du patient. La position du patient relativement au référentiel de l'installation d'imagerie détermine le point de vue de la représentation du système ostéo-articulaire sur chacune de ces images.

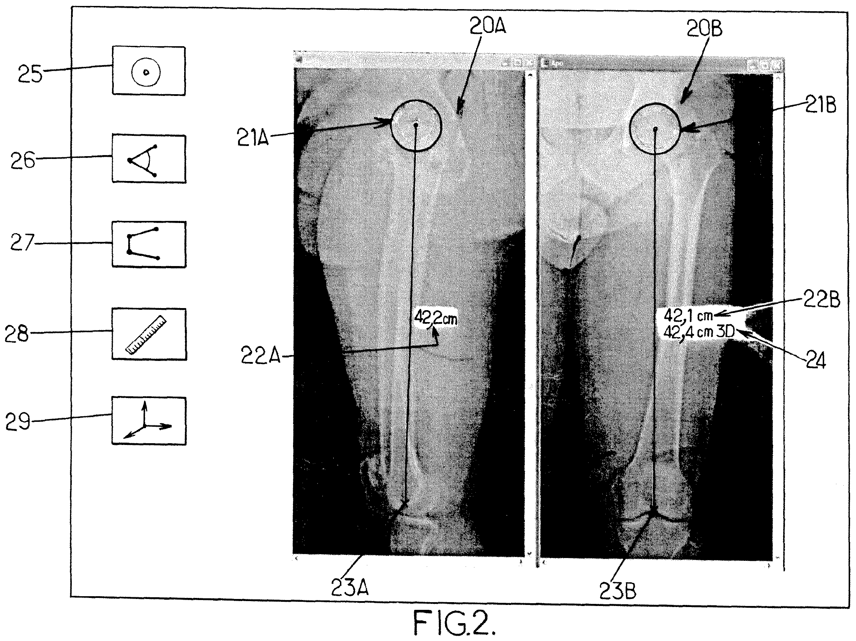

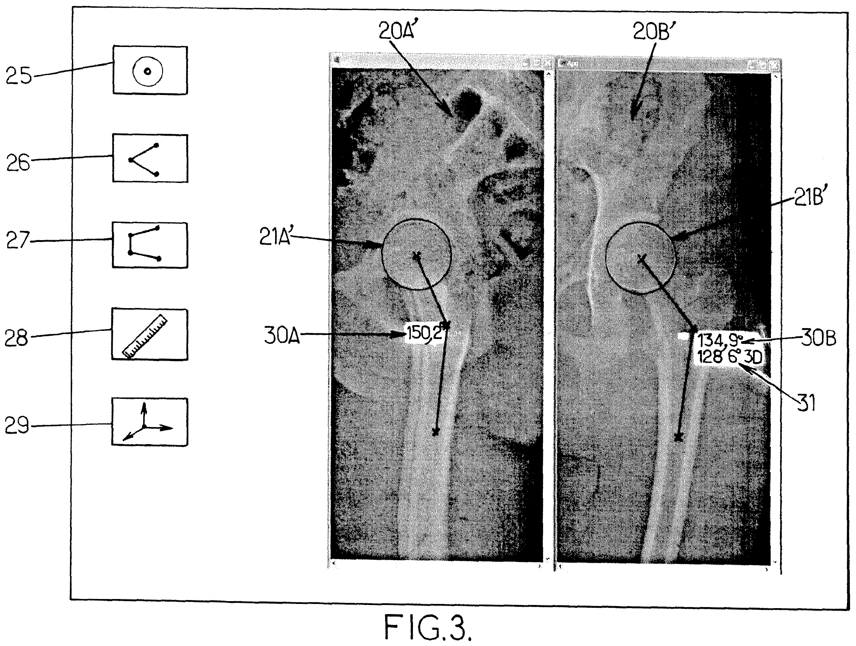

Les figures 2 et 3 montrent un exemple d'interface graphique affichable sur l'écran 19 de l'ordinateur 37, ou bien sur un autre écran. Sur ces deux figures, l'interface graphique est identique ; seules varient les images stéréoscopiques affichées et les grandeurs mesurées.

Cette interface graphique permet d'afficher les images stéréoscopiques 20A, 20B, 20A', 20B'.

Figurent également des icones 25, 26, 27, 28, 29. Chacune des icônes 25, 26, 27, 28 correspond à un type de grandeur géométrique, par exemple angle 3 points, angle 4 points, distance. A l'icône 25 peut correspondre une grandeur de rayon ou diamètre de sphère.

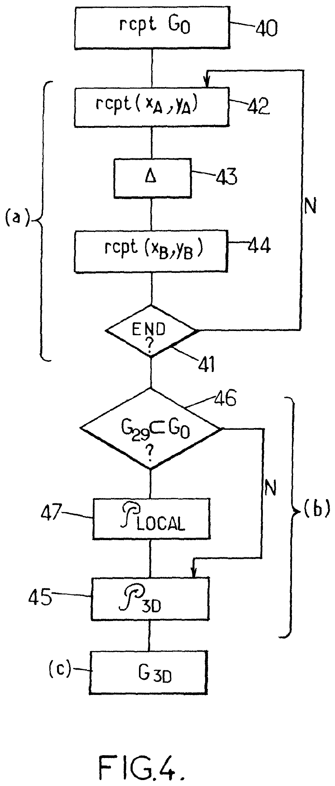

La figure 4 montre un exemple d'algorithme de mesure orthopédique de grandeurs géométriques intrinsèques à un système ostéo-articulaire, à partir de deux vues stéréoscopiques géométriquement calibrées de ce système ostéo-articulaire. Ces deux images peuvent être obtenues en utilisant le système de la figure 1, et être affichées sur l'interface graphique des figures 2 et 3. Les figures 2, 3 et 4 seront commentées simultanément.

L'utilisateur peut commencer par cliquer sur une des icônes 25 à 29, de sorte que le processeur (référence 17 sur la figure 1) reçoit lors d'une étape 40 une information relative au type de grandeur géométrique que l'utilisateur souhaite mesurer.

L'utilisateur sélectionne ensuite certains points sur les images 20A, 20B, 20A', 20B'. Le dispositif reçoit ainsi, lors d'une étape (a) des données de repérage comprenant des jeux de coordonnées bidimensionnelles, chaque jeu de coordonnées bidimensionnelles comprenant deux couples de coordonnées bidimensionnelles correspondant à deux points sur respectivement l'une et l'autre des images stéréoscopiques.

Le nombre de jeux de coordonnées reçu varie selon l'icône sélectionnée. Le test de fin d'acquisition 41 varie donc selon l'information reçue à l'étape 40.

Par exemple, si l'utilisateur a sélectionné l'icône 26 correspondant à une grandeur d'angle 3 points, il est mis fin à cette étape (a) d'acquisition lorsque trois jeux de coordonnées sont reçus. Le test 41 consiste donc à comparer le nombre de jeux de coordonnées reçu à un seuil.

Par exemple, si l'utilisateur a sélectionné l'icône 27 correspondant à une grandeur d'angle 4 points, il est mis fin à cette étape (a) d'acquisition lorsque quatre jeux de coordonnées sont reçus.

Par exemple, si l'utilisateur a sélectionné l'icône 28 correspondant à une grandeur de distance, il est mis fin à cette étape (a) d'acquisition lorsque deux jeux de coordonnées sont reçus.

Par exemple, si l'utilisateur a sélectionné l'icône 25 correspondant à une grandeur relative à une sphère, l'utilisateur peut, pour positionner deux cercles correspondant à la sphère respectivement sur les deux images, d'une part déplacer ces cercles, et d'autre part étirer ces cercles. Le dispositif est donc susceptible de recevoir un nombre de jeux de coordonnées qui peut ne pas être prédéfini. On peut prévoir que l'utilisateur clique sur une icône non représentée, ou sur une autre partie de l'interface graphique, pour valider les positions des cercles. Le test 41 peut alors consister à détecter ce clic.

Avantageusement, pour chaque jeu de coordonnées reçu, chaque couple de coordonnées se trouve sur un lieu des possibles de l'image correspondante, ce lieu des possibles étant déterminable à partir de l'autre couple de coordonnées et d'informations de calibration géométrique entre les images. Dit autrement, compte tenu des positions relatives des sources (références 5, 11 sur la figure 1, ou S_{A}, S_{B} sur la figure 5) et des détecteurs (références 6, 12 sur la figure 1, ou D_{A}, D_{B} sur la figure 5), on choisit des jeux de coordonnées définissant des points de l'espace 3D.

En particulier, on peut par exemple prévoir une étape 42 de réception d'un premier couple de coordonnées x_{A}, y_{A}, c'est-à-dire que l'utilisateur commence par sélectionner un point sur l'une 20A des images, dite première image.

Est déterminée lors d'une étape 43 un lieu des possibles Δ sur l'autre image 20B, dite deuxième image. La figure 5 illustre cette détermination du lieu des possibles. On définit une droite Δ_{3D} dans l'espace 3D entre un point P_{A} sur le détecteur D_{A} correspondant aux coordonnées x_{A}, y_{A} sur la première image et un point S_{A} de l'espace 3D correspondant à la source. Puis on simule une prise d'image de cette droite Δ_{3D} avec l'autre source (correspondant au point S_{B}) et l'autre détecteur (correspondant au point D_{B}). Le lieu des possibles Δ correspond donc à l'image simulée de cette droite Δ_{3D} sur l'autre image 20B. Chaque point de ce lieu des possibles Δ de l'image 20B définit avec le point correspondant aux coordonnées x_{A}, y_{A} sur l'image 20A un point de l'espace 3D.

Ce lieu des possibles Δ est affiché sur la deuxième image 20B.

L'utilisateur clique ensuite sur un point de ce lieu des possibles sur la deuxième image 20B, de sorte que le dispositif reçoit, lors d'une étape 44, un deuxième couple de coordonnées x_{B}, y_{B}.

Les étapes 42, 43, 44 représentent un algorithme d'acquisition de données de repérage relativement simple. On peut prévoir des étapes supplémentaires non représentées pour laisser à l'utilisateur plus de liberté pour fixer les jeux de coordonnées. Par exemple, on pourrait laisser à l'utilisateur le choix de cliquer sur la deuxième image ailleurs que sur le lieu des possibles défini à l'étape 43. Serait alors déterminé un lieu des possibles sur la première image, défini par le nouveau point cliqué. Le jeu de coordonnées pourrait ainsi être défini par des sélections successives.

Bien entendu, on pourrait prévoir de ne pas afficher un lieu des possibles, mais un seul point de ce lieu des possibles, que l'utilisateur déplacerait à sa guise sur l'interface graphique.

Alternativement, on peut prévoir de laisser l'utilisateur choisir deux points sur les images 20A, 20B, sans imposer une quelconque contrainte, puis, en utilisant les informations de calibration, de déterminer à partir des coordonnées de ces deux points saisis un jeu de coordonnées qui correspondrait à un point de l'espace 3D et qui serait relativement proche du jeu de coordonnées saisi par le praticien.

Suit une étape (b) au cours de laquelle est déterminée, en utilisant des informations de calibration géométrique, au moins une primitive géométrique tridimensionnelle définie par au moins une partie des données de repérage reçues à l'étape (a).

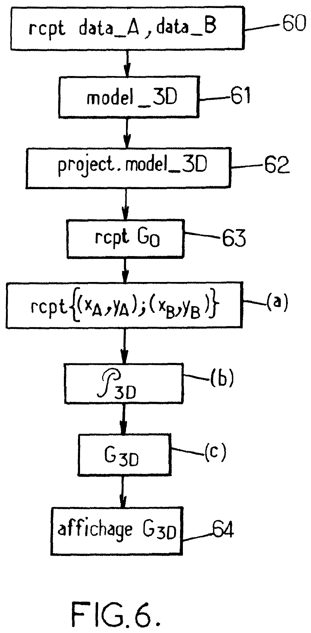

Cette détermination peut être basée sur les mêmes principes géométriques que la détermination d'un lieu des possibles. En effet, chaque couple de coordonnées obtenu correspond à un couple de points P_{A}, P_{B} sur la figure 6, et à un point P_{3D} de l'espace 3D. Ce point de l'espace 3D peut être déterminé relativement facilement, en utilisant par exemple les positions relatives des sources et des détecteurs. Et une fois le ou les point (s) de l'espace 3D déterminés, il est relativement aisé à l'homme du métier d'obtenir des primitives géométriques du type droites, sphères, ou autre.

Par exemple, si l'utilisateur a choisi de mesurer un angle 3 points, les trois jeux de coordonnées reçus à l'étape (a) permettent de définir, en utilisant les informations de calibration, trois points de l'espace 3D. Ces trois points de l'espace 3D permettent de définir deux droites concourant en un point (étape 45).

Par exemple, si l'utilisateur a choisi de mesurer un angle 4 points, les quatre jeux de coordonnées reçus à l'étape (a) permettent de définir deux droites de l'espace 3D lors de l'étape 45.

Par exemple, si l'utilisateur a choisi de mesurer une distance, les deux jeux de coordonnées reçus à l'étape (a) permettent de définir deux points de l'espace 3D, ou un segment de droite de l'espace 3D lors de l'étape 45.

Par exemple, si l'utilisateur a cliqué sur l'icône « sphère », les jeux de coordonnées reçus permettent de définir une sphère de l'espace 3D lors de l'étape 45.

Enfin, lors d'une étape (c), on calcule à partir d'au moins une primitive géométrique tridimensionnelle déterminée à l'étape (b) une valeur de grandeur géométrique intrinsèque au système ostéo-articulaire des deux images stéréoscopiques.

Par exemple, si l'utilisateur a choisi de mesurer un angle 3 points ou un angle 4 points, on calcule à partir des deux droites définies lors de l'étape 45 un angle entre ces deux droites.

Par exemple, si l'utilisateur a choisi de mesurer une distance, on calcule la distance dans l'espace 3D entre les deux points de l'espace 3D déterminés lors de l'étape 45.

Si l'utilisateur a cliqué sur l'icône « sphère », on détermine à partir de la sphère définie à l'étape 45 un rayon de la sphère par exemple.

En référence à la figure 2, l'utilisateur a cliqué sur l'icône 25 et ajusté deux cercles 21A, 21B sur les deux images 20A, 20B, de façon à sensiblement coïncider avec les représentations des condyles sur ces images. Lors de l'étape (b) une sphère est définie dans l'espace 3D à partir de ces cercles ajustés 21A, 21B.

Le rayon de la sphère peut être déterminé lors de l'étape (c) et peut fournir une information quant à la taille du condyle du patient, pour par exemple des applications de fabrication de prothèses.

La sphère définie à l'étape (b) peut servir à déterminer la position dans l'espace 3D de son centre. En effet, il peut être délicat pour le praticien de repérer le centre d'un condyle par simples pointages de points. En passant par une primitive sphère, le praticien utilise les représentations des bords des condyles sur les images pour déterminer ce centre.

La position dans l'espace 3D de ce centre, obtenue suite à l'étape (b) de définition de la primitive sphère, peut elle-même être utilisée pour définir d'autres primitives géométriques.

Par exemple, sur la figure 2, on cherche à mesurer une distance dans l'espace 3D entre le centre du condyle et l'autre extrémité du fémur. Un point de l'espace 3D correspondant à cette autre extrémité est défini à l'étape (b), à partir d'un jeu de coordonnées reçu à l'étape (a) correspondant aux points 23A, 23B.

Lors de l'étape (c) est calculée une distance dans l'espace 3D entre ces deux points de l'espace 3D.

Dans l'exemple représenté, on obtient une distance dans l'espace 3D de 42,4 cm (référence 24). Les distances 22A, 22B mesurées directement dans les plans des images, entre les centres des cercles 20A, 20B et les points 23A, 23B respectivement sont de 42,2 cm et 42,1 cm.

Dans l'exemple de la figure 3, le praticien a également utilisé une primitive sphère pour déterminer un point de l'espace 3D correspondant au centre d'un condyle. Deux autres points de l'espace 3D sont définis à partir de deux jeux de coordonnées reçus à l'étape (a). Lors de l'étape (c) est calculé un angle dans l'espace 3D à partir des positions dans l'espace 3d de ces trois points.

Dans l'exemple représenté, on obtient un angle dans l'espace 3D de 128,6°, tandis que les angles mesurés dans les plans des images sont de 150,2° pour l'image 20A' et 134,9° pour l'image 20B'.

L'interface graphique des figures 2 et 3 comporte une icône 29, dite icône de repère local. Cette icône permet de définir un repère local, à partir de jeux de coordonnées saisis par le praticien.

Lorsque le praticien clique sur cette icône, il manipule des représentations en deux dimensions d'un repère tridimensionnel, de la même manière qu'il manipule des cercles lorsqu'il clique sur l'icône 25. Le praticien peut donc positionner et orienter ces représentations du repère local, de façon à ce que puisse être défini lors de l'étape (b) un repère local dans l'espace 3D, à partir des informations de calibration géométrique et à partir de jeux de coordonnées saisis par le praticien lors des positionnements/orientations.

Ce repère local défini dans l'espace 3D permet de définir en particulier au moins un plan de l'espace 3D, par exemple le plan XY du repère local et/ou le plan XZ.

Par exemple, l'étape (b) comporte une étape de test 46 : si certaines des données de repérage reçues à l'étape (a) correspondent à un repère 3D, un repère local est défini lors d'une étape 47.

Il peut être envisagé, lors de l'étape (c) de calcul d'une grandeur géométrique, de projeter tout ou partie des primitives obtenues à l'étape (b) autres que ce repère 3D dans un ou plusieurs plans du repère 3D.

Par exemple, les droites définies dans l'espace 3D lors de l'étape (b) sont projetées dans l'un des plans également défini à l'étape (b).

On calcule ensuite une ou des valeurs de grandeurs géométriques à partir de cette ou ces projections dans un ou plusieurs plans du repère local.

Par exemple, on calcule un angle dans le plan du repère local entre les projetés dans ce plan de deux droites définies à l'étape (b).

Par exemple, on calcule une distance dans le plan du repère local entre les projetés dans ce plan de deux points de l'espace 3D, ou bien entre les projetés dans ce plan d'un point de l'espace 3D et d'une droite de l'espace 3D.

En variante, le repère local défini dans l'espace 3D permet de définir en particulier au moins un axe de l'espace 3D, par exemple l'axe X du repère local, l'axe Y et/ou l'axe Z. Il peut être envisagé, lors de l'étape (c) de calcul d'une grandeur géométrique, de projeter tout ou partie des primitives obtenues à l'étape (b) autres que ce repère 3D dans un ou plusieurs axes du repère 3D.

La figure 6 montre un autre algorithme, dans lequel deux ensembles de données d'imagerie sont reçus lors d'une étape 60. Deux images stéréoscopiques obtenues à partir de ces ensembles sont affichées. Le praticien peut repérer certains points particuliers sur l'une et l'autre des images. Un modèle 3D est reconstruit à partir de ces images, d'un modèle a priori de la structure à reconstruire, et des points particuliers appariés d'une image à l'autre (étape 61).

Puis le modèle obtenu est projeté sur deux plans non parallèles, par exemple définis par le praticien, lors d'une étape 62.

Le praticien peut alors souhaiter effectuer des mesures de grandeurs géométriques intrinsèques au système ostéo-articulaire du patient. Le praticien clique sur l'une des icônes 25 à 29, de sorte qu'une indication du type de grandeur que l'on souhaite mesurer est reçue lors d'une étape 62. Des données de repérage sont reçues lors d'une étape (a), des primitives géométriques 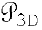 sont déterminées lors d'une étape (b) et une grandeur G_{3D} est calculée à partir de ces primitives 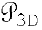 , lors d'une étape (c). Cette grandeur G_{3D} est affichée sur un écran lors d'une étape 64.

## Revendications

1. Procédé de mesure de grandeurs géométriques intrinsèques à un système anatomique, à partir de deux images bidimensionnelles stéréoscopiques géométriquement calibrées du système anatomique d'un patient, comprenant
(a) recevoir des données de repérage comprenant plusieurs jeux de coordonnées bidimensionnelles, chaque jeu de coordonnées bidimensionnelles comprenant
un premier couple (x_{A}, y_{A}) de coordonnées bidimensionnelles correspondant à un premier point sur l'une desdites deux images stéréoscopiques et
un deuxième couple (x_{B}, y_{B}) de coordonnées bidimensionnelles correspondant à un deuxième point sur l'autre desdites deux images stéréoscopiques,
(b) en utilisant des informations de calibration géométrique, déterminer au moins une primitive géométrique tridimensionnelle ( 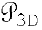 ) définie par au moins une partie des données de repérage reçues à l'étape (a),
(c) calculer à partir d'au moins une primitive géométrique tridimensionnelle déterminée à l'étape (b) une valeur de grandeur géométrique (G_{3D}) intrinsèque au système anatomique,
dans lequel
à l'étape (c), on calcule au moins une valeur de grandeur géométrique dans au moins un plan, à partir d'au moins un projeté sur ledit au moins un plan d'au moins une primitive géométrique tridimensionnelle déterminée à l'étape (b), ledit plan étant défini à partir de données de repérage reçues à l'étape (a),
dans lequel une primitive géométrique tridimensionnelle est une sphère, une droite, un cylindre, un cône, ou un pavé,
et dans lequel la grandeur géométrique est un angle ou une aire.

2. Procédé de mesure selon la revendication 1, dans lequel les deux images stéréoscopiques sont obtenues par deux projections (62) respectives d'un modèle tridimensionnel du système ostéo-articulaire du patient selon des directions distinctes sur des plans virtuels simulant des plans d'acquisition.

3. Procédé de mesure selon la revendication 1, dans lequel les deux images stéréoscopiques sont reçues de deux détecteurs à rayons X respectifs.

4. Procédé de mesure selon l'une quelconque des revendications précédentes, dans lequel, à l'étape (a), les données de repérage sont reçues suite à la sélection, par un utilisateur, de points sur les images à l'aide d'une interface utilisateur.

5. Procédé de mesure selon l'une quelconque des revendications précédentes, comprenant en outre une étape d'affichage sur un écran de la valeur de grandeur géométrique calculée à l'étape (c).

6. Procédé de mesure selon la revendication 5, dans lequel, suite à l'étape d'affichage, les étapes (a), (b), (c) et l'étape d'affichage sont répétées.

7. Procédé de mesure selon l'une quelconque des revendications précédentes, dans lequel les données de repérage reçues à l'étape (a) comprennent trois ou quatre jeux de coordonnées bidimensionnelles.

8. Procédé de mesure selon la revendication précédente, dans lequel la grandeur géométrique dont la valeur est calculée à l'étape (c) est un angle.

9. Programme d'ordinateur comportant des instructions pour la mise en œuvre du procédé selon l'une des revendications précédentes, lorsque lesdites instructions sont exécutées par un processeur.

10. Dispositif (17) de mesure de grandeurs géométriques intrinsèques à un système anatomique, à partir de deux images bidimensionnelles stéréoscopiques calibrées géométriquement du système anatomique d'un patient, comprenant
(a) des moyens de réception de données de repérage comprenant plusieurs jeux de coordonnées bidimensionnelles, chaque jeu de coordonnées bidimensionnelles comprenant un premier couple de coordonnées bidimensionnelles correspondant à un premier point sur l'une desdites deux images stéréoscopiques et un deuxième couple de coordonnées bidimensionnelles correspondant à un deuxième point sur l'autre desdites deux images stéréoscopiques,
(b) des moyens de traitement agencés pour
en utilisant les informations de calibration géométrique, déterminer au moins une primitive géométrique tridimensionnelle définie par au moins une partie des données de repérage reçues par les moyens de réception, et pour
calculer à partir d'au moins une primitive géométrique tridimensionnelle ainsi déterminée une valeur de grandeur géométrique intrinsèque au système anatomique,
une primitive géométrique tridimensionnelle étant une sphère, une droite, un cylindre, un cône, ou un pavé, et de sorte qu'on calcule au moins une valeur de grandeur géométrique dans au moins un plan, à partir d'au moins un projeté sur ledit au moins un plan d'au moins une primitive géométrique tridimensionnelle déterminée, ledit plan étant défini à partir de données de repérage reçues,
la grandeur géométrique étant un angle ou une aire.

## Patentansprüche

1. Messverfahren für geometrische Größen, die intrinsisch für ein anatomisches System sind, ausgehend von zwei geometrisch kalibrierten, zweidimensionalen, stereoskopischen Bildern des anatomischen Systems eines Patienten, umfassend:
(a) Empfangen von Ortsdaten, die mehrere Sätze zweidimensionaler Koordinaten umfassen, wobei jeder Satz zweidimensionaler Koordinaten ein erstes Paar (x_{A}, y_{A}) zweidimensionaler Koordinaten umfasst, das einem ersten Punkt auf einem der beiden stereoskopischen Bilder entspricht, und
ein zweites Paar (x_{B}, y_{B}) zweidimensionaler Koordinaten umfasst, das einem zweiten Punkt auf dem anderen der beiden stereoskopischen Bilder entspricht,
(b) Bestimmen mindestens einer dreidimensionalen geometrischen Grundform ( 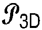 ), welche durch wenigstens einen Teil der in Schritt (a) empfangenen Ortsdaten definiert wird, unter Verwendung von geometrischen Kalibrierungsinformationen,
(c) Berechnen eines Wertes der für das anatomische System intrinsischen geometrischen Größe (G_{3D}) auf Grundlage von wenigstens einer im Schritt (b) bestimmten dreidimensionalen geometrischen Grundform, wobei in Schritt (c) mindestens ein Wert der geometrischen Größe in mindestens einer Ebene berechnet wird, ausgehend von mindestens einer Projektion mindestens einer in Schritt (b) bestimmten dreidimensionalen geometrischen Grundform auf die mindestens eine Ebene, wobei die Ebene auf Grundlage von in Schritt (a) empfangenen Ortsdaten definiert wird, wobei eine dreidimensionale geometrische Grundform eine Kugel, eine Gerade, ein Zylinder, ein Kegel oder ein Klotz ist,
und wobei die geometrische Größe ein Winkel oder ein Flächeninhalt ist.

2. Messverfahren nach Anspruch 1, wobei die beiden stereoskopischen Bilder durch jeweils zwei Projektionen (62) eines dreidimensionalen Modells des Osteo-Gelenk-Systems des Patienten in unterschiedlichen Richtungen auf virtuelle Ebenen erhalten werden, die Aufnahme-Ebenen simulieren.

3. Messverfahren nach Anspruch 1, wobei die beiden stereoskopischen Bilder von zwei jeweiligen Röntgendetektoren empfangen werden.

4. Messverfahren nach einem der vorhergehenden Ansprüche, bei dem in Schritt (a) die Ortsdaten empfangen werden, nachdem ein Benutzer mit Hilfe einer Benutzerschnittstelle Punkte auf den Bildern ausgewählt hat.

5. Messverfahren nach einem der vorhergehenden Ansprüche, ferner umfassend einen Schritt des Anzeigens des in Schritt (c) berechneten Werts der geometrischen Größe auf einem Bildschirm.

6. Messverfahren nach Anspruch 5, bei dem nach dem Schritt des Anzeigens die Schritte (a), (b), (c) und der Schritt des Anzeigens wiederholt werden.

7. Messverfahren nach einem der vorhergehenden Ansprüche, wobei die in Schritt (a) empfangenen Ortsdaten drei oder vier Sätze zweidimensionaler Koordinaten umfassen.

8. Messverfahren nach dem vorhergehenden Anspruch, wobei die geometrische Größe, deren Wert in Schritt (c) berechnet wird, ein Winkel ist.

9. Computerprogramm mit Anweisungen zur Durchführung des Verfahrens nach einem der vorhergehenden Ansprüche, wenn die Anweisungen von einem Prozessor ausgeführt werden.

10. Vorrichtung (17) zum Messen geometrischer Größen, die intrinsisch für ein anatomisches System sind, ausgehend von zwei geometrisch kalibrierten, stereoskopischen, zweidimensionalen Bildern des anatomischen Systems eines Patienten, umfassend:
(a) Empfangsmittel für Ortsdaten, die mehrere Sätze von zweidimensionalen Koordinaten umfassen, wobei jeder Satz von zweidimensionalen Koordinaten ein erstes Paar von zweidimensionalen Koordinaten umfasst, das einem ersten Punkt auf einem der beiden stereoskopischen Bilder entspricht, und ein zweites Paar von zweidimensionalen Koordinaten umfasst, das einem zweiten Punkt auf dem anderen der beiden stereoskopischen Bilder entspricht,
(b) Verarbeitungsmittel, die dazu ausgelegt sind, unter Verwendung von geometrischen Kalibrierungsinformationen mindestens eine dreidimensionale geometrische Grundform zu bestimmen, die durch mindestens einen Teil der von den Empfangsmitteln empfangenen Ortsdaten definiert ist,
und dazu, ausgehend von mindestens einer so bestimmten dreidimensionalen geometrischen Grundform einen Wert der geometrischen Größe zu berechnen, die intrinsisch für das anatomische System ist,
wobei eine dreidimensionale geometrische Grundform eine Kugel, eine Gerade, ein Zylinder, ein Kegel oder ein Klotz ist,
und so dass mindestens ein Wert der geometrischen Größe in mindestens einer Ebene berechnet wird, ausgehend von mindestens einer Projektion mindestens einer bestimmten dreidimensionalen geometrischen Grundform auf die mindestens eine Ebene, wobei die Ebene auf der Grundlage von empfangenen Ortsdaten definiert ist,
wobei die geometrische Größe ein Winkel oder ein Flächeninhalt ist.

## Claims

1. Method for measuring intrinsic geometric quantities in an anatomical system, based on two geometrically calibrated stereoscopic two-dimensional images of a patient's anatomical system, comprising
(a) receiving location data comprising a plurality of sets of two-dimensional coordinates, each set of two-dimensional coordinates comprising a first pair (x_{A}, y_{A}) of two-dimensional coordinates corresponding to a first point on one of said two stereoscopic images and
a second pair (x_{B}, y_{B}) of two-dimensional coordinates corresponding to a second point on the other of said two stereoscopic images,
(b) using geometric calibration data, determining at least one three-dimensional geometric primitive ( 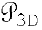 ) defined by at least a portion of the location data received in step (a),
(c) computing based on at least one three-dimensional geometric primitive determined in step (b) an intrinsic geometric quantity value (G_{3D}) in the anatomical system,
wherein
in step (c), at least one geometric quantity value is computed in at least one plane, based on at least one projection onto said at least one plane of at least one three-dimensional geometric primitive determined in step (b), said plane being defined based on location data received in step (a),
wherein a three-dimensional geometric primitive is a sphere, a line, a cylinder, a cone, or a block,
and wherein the geometric quantity is an angle or an area.

2. Measurement method according to claim 1, wherein the two stereoscopic images are obtained by two respective projections (62) of a three-dimensional model of the patient's osteoarticular system along different directions on virtual planes simulating acquisition planes.

3. Measurement method according to claim 1, wherein the two stereoscopic images are received from two respective X-ray detectors.

4. Measurement method according to any one of the preceding claims, wherein, in step (a), the location data are received following the selection, by a user, of points on the images using a user interface.

5. Measurement method according to any one of the preceding claims, further comprising a step of displaying the geometric quantity value computed in step (c) on a screen.

6. Measurement method according to claim 5, wherein, following the display step, steps (a), (b), (c) and the display step are repeated.

7. Measurement method according to any one of the preceding claims, wherein the location data received in step (a) comprise three or four sets of two-dimensional coordinates.

8. Measurement method according to the preceding claim, wherein the geometric quantity, the value whereof is computed in step (c), is an angle.

9. Computer program including instructions for implementing the method according to one of the preceding claims, when said instructions are executed by a processor.

10. Device (17) for measuring intrinsic geometric quantities in an anatomical system, based on two geometrically calibrated stereoscopic two-dimensional images of a patient's anatomical system, comprising
(a) means for receiving location data comprising a plurality of sets of two-dimensional coordinates, each set of two-dimensional coordinates comprising a first pair of two-dimensional coordinates corresponding to a first point on one of said two stereoscopic images and a second pair of two-dimensional coordinates corresponding to a second point on the other of said two stereoscopic images,
(b) processing means arranged to, using geometric calibration data, determine at least one three-dimensional geometric primitive defined by at least a portion of the location data received by the receiving means, and to compute based on at least one three-dimensional geometric primitive thus determined an intrinsic geometric quantity value in the anatomical system, a three-dimensional geometric primitive being a sphere, a line, a cylinder, a cone, or a block,
and such that at least one geometric quantity value is computed in at least one plane, based on at least one projection onto said at least one plane of at least one determined three-dimensional geometric primitive, said plane being defined based on received location data, the geometric quantity being an angle or an area.
